# EUROPEAN PATENT APPLICATION

(11) **EP 4 406 495 A1**
(43) Date of publication of application: **31.07.2024**
(21) Application number: 22872077.7
(22) Date of filing: 22.09.2022
(51) Int. Cl.: A61B 17/072, A61B 17/3209

(54) **STAPLE MAGAZINE ASSEMBLY AND SURGICAL INSTRUMENT**

(30) Priority: 23.09.2021 CN 202111114909; 23.09.2021 CN 202122299036 U
(71) Applicant: Fulbright Medical Inc., Wuxi, Jiangsu 214437 (CN)
(72) Inventor: SUN, Baofeng, Wuxi, Jiangsu 214437 (CN); SHI, Longxia, Wuxi, Jiangsu 214437 (CN)
(74) Representative: Maiwald GmbH
(86) International application number: PCT/CN2022/120654
(87) International publication number: WO 2023/046033

(57) **Abstract**

A staple magazine assembly (101) and a surgical instrument, the staple magazine assembly (101) comprising a staple magazine body (3) and a hammer plate assembly (1) mounted on the staple magazine body (3), the hammer plate assembly (1) comprising: a hammer plate (11), the hammer plate (11) being provided with a blade receiving groove (110), the blade receiving groove (110) being provided with a first guide member (112), and also a cutting blade (12), the cutting blade (12) being at least partially accommodated in the blade receiving groove (110); the cutting blade (12) is provided with a second guide member (121), and one of the first guide member (112) and the second guide member (121) extends in a direction perpendicular to a staple output surface (31) of the staple magazine body (3); the first guide member (112) and the second guide member (121) are movably mated relative to one another, and can guide the cutting blade (12) to move in a direction perpendicular to the staple output surface (31).

## Description

### RELATED APPLICATIONS

The present application claims the priority of a Chinese patent application submitted to the China National Intellectual Property Administration on September 23, 2021, with application number 202111114909.6 and application title "staple cartridge assembly and surgical instrument", and its entire content is incorporated in this application by reference.

### TECHNICAL FIELD

The present application belongs to the field of medical surgical instruments, particularly relates to a staple cartridge assembly and a surgical instrument.

### BACKGROUND

Surgical instruments can simultaneously perform anastomosis tasks and cutting tasks, reduce surgical time, and reduce patient bleeding, which play a crucial role in clinical surgery. Cutting knives are essential components of surgical instruments. The front end of a surgical instrument includes a replaceable staple cartridge assembly, which is a disposable consumable.

In order to avoid wear and poor cutting effect caused by repeated use of cutting knives, staple cartridge assemblies integrated with cutting knives have emerged. This type of staple cartridge assembly hides the cutting knife inside the staple cartridge body to prevent the sharp blade from harming the user when not installed on the surgical instrument. When this type of staple cartridge assembly is installed on a surgical instrument, the blade of the cutting knife extends out of the surface of the staple cartridge body, that is, blade protrusion, to cut tissue during the surgical process.

The cutting knife of the staple cartridge assembly in the prior art can slide along the inclined guiding groove under the action of external force to switch between the hidden state and the blade protrusion state mentioned above. The inclined guiding groove leads to a longer length of a pushing staple board, affecting the strength of the pushing staple board and resulting in a longer length of the staple cartridge, which is not conducive to surgical operations in limited space.

### SUMMARY

The present application aims to provide a staple cartridge assembly and a surgical instrument to solve the technical problem of the inclined guiding groove of the cutting knife in the prior art affecting the length of the staple cartridge assembly.

The present application provides a staple cartridge assembly, the staple cartridge assembly includes a staple cartridge body and a pushing staple board assembly installed on the staple cartridge body, the pushing staple board assembly includes:

a pushing staple board, in which the pushing staple board is provided with a knife receiving groove, the knife receiving groove is provided with a first guiding member, and a cutting knife, in which the cutting knife is at least partially accommodated in the knife receiving groove, and the cutting knife is provided with a second guiding member; one of the first guiding member and the second guiding member extends along a direction perpendicular to a staple outlet surface of the staple cartridge body, the first guiding member and the second guiding member are connected in a relatively movable manner, and the first guiding member and the second guiding member are capable of guiding the cutting knife to move along a direction perpendicular to the staple outlet surface.

Preferably, the cutting knife includes a first surface, the first surface is configured to be pushed to allow the cutting knife to move upwards relative to the pushing staple board along the direction perpendicular to the staple outlet surface under a guidance of the first guiding member and the second guiding member, to allow a blade of the cutting knife to protrude from the staple outlet surface.

Preferably, after the cutting knife moves upward in the direction perpendicular to the staple outlet surface to allow the blade of the cutting knife to protrude from the staple outlet surface, the first surface is contacted to maintain the blade of the cutting knife protruding from the staple outlet surface.

Preferably, the first surface is a plane parallel to the staple outlet surface.

Preferably, the knife receiving groove is provided with an opening, the opening allows the first surface to be exposed from the knife receiving groove and to be contactable.

Preferably, the cutting knife includes a second surface, at least a part of the second surface is a plane inclined relative to the staple outlet surface or an arc-shaped surface inclined and protruding outward relative to the staple outlet surface, the second surface faces rear and lower sides of the pushing staple board, the second surface is configured to be pushed to allow the cutting knife to move upwards in the direction perpendicular to the staple outlet surface under a guidance of the first guiding member and the second guiding member, to allow a blade of the cutting knife to protrude from the staple outlet surface.

Preferably, a first surface of the cutting knife is connected with the second surface, and the first surface is located on a front side of the second surface; the first surface is contacted to maintain the blade of the cutting knife protruding from the staple outlet surface.

Preferably, the first surface is a plane parallel to the staple outlet surface.

Preferably, the cutting knife further includes a second surface, at least a part of the second surface is a surface inclined relative to the staple outlet surface or an arc-shaped surface inclined and protruding outward relative to the staple outlet surface, the second surface faces rear and lower sides of the pushing staple board, the second surface is configured to be pushed to allow the cutting knife to move upwards in the direction perpendicular to the staple outlet surface under a guidance of the first guiding member and the second guiding member, to allow the blade of the cutting knife to protrude from the staple outlet surface; the first surface is connected with the second surface, and the first surface is located on a front side of the second surface.

Preferably, the knife receiving groove is provided with at least one set of first guiding members, and one set of first guide members includes two first guiding members located respectively on opposite side walls of the knife receiving groove, the cutting knife is provided with at least one set of second guide members, one set of second guide members includes two second guiding members located respectively on opposite sides of the cutting knife, the two first guiding members of the one set of first guiding members are matched with the two second guiding members of the one set of second guiding members one by one.

Preferably, the knife receiving groove is provided with two sets of first guiding members, the two sets of first guiding members are spaced apart in an extension direction of the staple outlet surface, the cutting knife is provided with two sets of second guiding members, the two sets of second guiding members are spaced apart in the extension direction of the staple outlet surface, a front set of first guiding members in the two sets of first guiding members is matched with a front set of second guiding members in the two sets of second guiding members, and a rear set of first guiding members in the two sets of first guiding members is matched with a rear set of second guiding members in the two sets of second guiding members.

Preferably, the pushing staple board assembly further includes an elastic body, one end of the elastic body is installed on the pushing staple board, the other end of the elastic body is against the cutting knife, and the elastic body is configured to drive the cutting knife to move downwards in the direction perpendicular to the staple outlet surface.

Preferably, the pushing staple board assembly further includes a support board, the knife receiving groove includes an accommodating chamber for accommodating at least a part of the elastic body, the accommodating chamber includes an opening, and the support board is installed on the pushing staple board to at least partially seal the opening.

The present application further provides a surgical instrument, the surgical instrument includes an end effector and a propeller, the end effector includes a jaw assembly and the above staple cartridge assembly, the staple cartridge assembly is detachably installed on the jaw assembly,

the propeller is configured to drive the pushing staple board to move forward along a length direction, the propeller includes a knife lifting part extending forward, the knife lifting part includes a third surface;

in a case that the staple cartridge assembly is installed on the jaw assembly, the knife lifting part is located below the cutting knife, and the third surface acts on the cutting knife from below the cutting knife, to allow a blade of the cutting knife to protrude from the staple outlet surface of the staple cartridge assembly.

Preferably, the third surface is a plane parallel to the staple outlet surface of the staple cartridge assembly installed on the jaw assembly.

Preferably, the knife lifting part further includes a fourth surface, the fourth surface is a surface inclined relative to the staple outlet surface of the staple cartridge assembly installed on the jaw assembly, or an arc-shaped surface inclined and recessed inwardly relative to the staple outlet surface, the fourth surface faces front and upper sides of the surgical instrument; in a case that the knife lifting part moves forward, the fourth surface is capable of contacting and pushing the cutting knife to slide relative to the fourth surface, thereby allowing the cutting knife to move upward in the direction perpendicular to the staple outlet surface.

Preferably, the third surface is connected with the fourth surface, and the fourth surface is located on a front side of the third surface.

Preferably, the propeller is provided with an arm portion, the arm portion and the knife lifting part are spaced apart, and a concave part is provided between the arm portion and the knife lifting part, the concave part is capable of accommodating the cutting knife (12).

Preferably, a guiding surface is provided at a front end of the arm portion, in a case that the cutting knife is accommodated in the concave part, the guiding surface protrudes forward from a tip of the cutting knife, the guiding surface faces the concave part, and the guiding surface is an inclined surface or an arc-shaped surface.

Preferably, the arm portion is provided with a groove, the groove faces the concave part, in a case that the cutting knife is accommodated in the concave part, an upper edge of the cutting knife is embedded in the groove.

Preferably, the third surface is configured to push the cutting knife from below the cutting knife, to allow the cutting knife to move in the direction perpendicular to the staple outlet surface, so that the blade of the cutting knife protrudes from the staple outlet surface of the staple cartridge assembly; or the third surface contacts the cutting knife from below the cutting knife to maintain the blade of the cutting knife protruding from the staple outlet surface.

By adopting the above technical solution, the cutting knife is guided by the first guiding component and the second guiding component to achieve vertical blade protrusion. During the blade protrusion process, the movement of the cutting knife does not utilize the space in the length direction inside the staple cartridge, making the staple cartridge assembly short while maintaining the same anastomosis stroke, making it easier to perform surgery.

### BRIEF DESCRIPTION OF THE DRAWINGS

FIG. 1 is a structural schematic diagram of a stapler according to an embodiment of the present application;
FIG. 2 is a structural schematic diagram of a jaw assembly of a stapler according to an embodiment of the present application;
FIG. 3 is a structural schematic diagram of a pushing staple board assembly of a stapler according to an embodiment of the present application;
FIG. 4 is a structural schematic diagram of the pushing staple board assembly of the stapler from another angle according to an embodiment of the present application;
FIG. 5 is a cross-sectional view of a pushing staple board assembly of a stapler according to an embodiment of the present application;
FIG. 6 is an exploded view of a pushing staple board assembly of a stapler according to an embodiment of the present application;
FIG. 7 is a structural schematic diagram of a propeller of a stapler according to an embodiment of the present application;
FIG. 8A is a partial enlarged sectional view of a propeller of a stapler according to an embodiment of the present application at a starting position;
FIG. 8B is a partial enlarged view of FIG. 8A;
FIG. 9A is a partial enlarged sectional view of a propeller of a stapler according to an embodiment of the present application at a certain position in the middle;
FIG. 9B is a partial enlarged view of FIG. 9A;
FIG. 10A is a partial enlarged cross-sectional view of a propeller of a stapler according to an embodiment of the present application when retracted;
FIG. 10B is a partial enlarged view of FIG. 10A;
FIG. 11A is a partial enlarged cross-sectional view of a propeller of a stapler according to an embodiment of the present application when it re-strikes after retraction;
FIG. 11B is a partial enlarged view of FIG. 11A;
FIG. 12A is a partial enlarged cross-sectional view of a situation where a staple cartridge assembly is installed on a staple cartridge holder;
FIG. 12B is a partial enlarged view of FIG. 12A;
FIG. 13A is a partial enlarged sectional view of another situation where a staple cartridge assembly is installed on a staple cartridge holder;
FIG. 13B is a partial enlarged view of FIG. 13A; and
FIG. 14 is a partial enlarged view of a possible presence of a staple cartridge assembly installed on a stapler.

Description of reference numerals: 100. end effector; 101. staple cartridge assembly; 200. sleeve assembly; 300. handle assembly; 10. staple cartridge holder; 20. staple support holder; 1. pushing staple board assembly; 11. pushing staple board; 110. knife receiving groove; 111. slot; 112. first guiding member; 113. accommodating chamber; 114. force bearing portion; 12. cutting knife; 121. second guiding member; 122. second surface; 123. first surface; 1201. knife body; 1202. retaining structure; 13. elastic body; 14. support board; 2. propeller; 21. knife holder; 22. knife holder head; 221. knife lifting part; 2211. fourth surface; 2212. third surface; 222. arm portion; 2221. guiding surface; 2222. groove; 223. concave part; 224. pushing portion; 3. staple cartridge body; 31. staple outlet surface; A. guiding groove; B. sliding block; X. length direction; Y width direction; Z. height direction.

### DETAILED DESCRIPTION

In order to illustrate the above purposes, features, and advantages of the present application more clearly, the specific embodiments of the present application will be described in detail in combination with the attached drawings in this section. In addition to various embodiments described in this section, the present application can further be implemented through other different ways. Without violating the spirit of the present application, those skilled in the art may make corresponding improvements, deformations and substitutions, and thus the present application is not limited by the specific embodiments disclosed in this section. The protection scope of the present application shall be based on the claims.

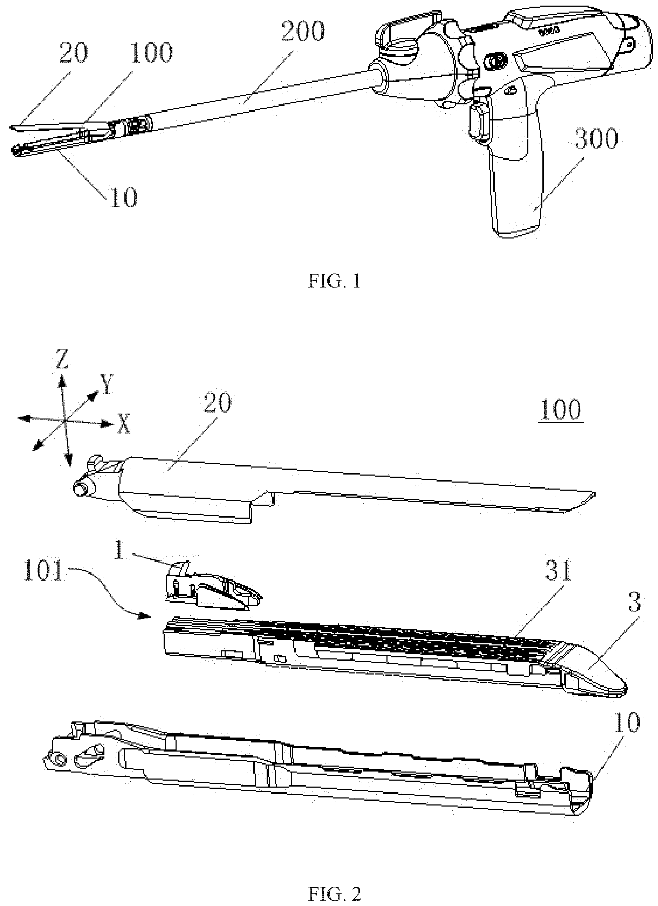

Users of the stapler may be clinical doctors who operate the stapler during surgery. The terms "near", "rear", "distal", and "front" used herein are relative to the clinical doctor who manipulate the stapler. The terms "near" and "rear" refer to the part relatively close to the clinical doctor, while the terms "distal" and "front" refer to the part relatively far away from the clinical doctor. "Left" and "right" refer to the position of the stapler shown in FIG. 1, for example, the end effector 100 is on "left", the handle assembly 300 and the shaft assembly are on "right". The terms "up/above" and "down/below" refer to the relative position of the staple support holder 20 and the staple cartridge holder 10 of the end effector 100. Specifically, the staple support holder 20 is located "up" and "above" the staple cartridge holder 10 and the staple cartridge holder 10 is located "down" and "below" the staple support holder 20. In the staple cartridge assembly 101, the staple outlet surface 31 is "up" and the side opposite to the staple outlet surface 31 is "down". When the staple cartridge assembly 101 is installed on the staple cartridge holder 10, the staple outlet surface 31 of the staple cartridge assembly 101 faces the staple support holder 20, that is, the staple outlet surface 31 of the staple cartridge assembly 101 faces "up".

If not specified separately, the length direction X represents the length direction of the staple cartridge assembly 101, and the length direction of the staple cartridge assembly 101 is consistent with the front and rear direction of the stapler. The width direction Y represents the width direction of the staple cartridge assembly 101, and the width direction Y is perpendicular to the length direction. The height direction Z represents the height direction of the staple cartridge assembly 101, and the height direction of the staple cartridge assembly 101 is consistent with the up and down direction of the stapler. The height direction Z is perpendicular to the length direction X and the width direction Y, specifically, as shown in FIG. 2. The height direction Z is consistent with the direction perpendicular to the staple outlet surface 31.

It should be understood that the directions "distal", "near", "front", "rear", "left", "right", "up/above", and "down/below" are defined for the convenience of description. However, the stapler can be used in many directions and positions. Therefore, these terms that express relative positional relationships are not restrictive or absolute.

In the present application, unless otherwise specified and limited, terms such as "connection", "connected", "setting", etc. should be broadly understood. For example, the connection may be a fixed connection, a detachable connection, a movable connection, or integration; it may be a direct connection or an indirect connection through an intermediate medium, and may be an internal connection between two components or an interaction relationship between two components. For those skilled in the art, the specific meanings of the above terms in this application can be understood according to specific situations.

That is to say, the definitions of various directions in this specification are only for the convenience of illustrating the technical solution of the present application, and do not limit the direction of the electric stapler in the embodiments of the present application in the scenarios including but not limited to manufacturing, testing, transportation, use, etc. that may cause the orientation of the electric stapler to be reversed or the position of the electric stapler to be changed.

The embodiments described below with reference to the attached drawings are exemplary and intended to explain the present application, but cannot be understood as limitations to the present application.

As shown in FIG. 1, the present application provides a surgical instrument, preferably a stapler, which includes an end effector 100, a sleeve assembly 200, and a handle assembly 300. The handle assembly 300 is connected to the end effector 100 through the sleeve assembly 200.

The handle assembly 300 includes a housing and a power source module. In one implementation, the power source module is a handle which is manually operated by the user and connected to the following transmission device. In another implementation, the power source module includes a motor and a power module arranged inside the housing. The power module is composed of multiple electrically connected batteries, which are used to provide the necessary electrical energy for the rotation of the motor. The motor is connected to the following transmission device. The type of the power source module does not limit the protection scope of the present application. In the following, the power source module including the motor and the power module is taken as an example to describe the overall functions of the stapler.

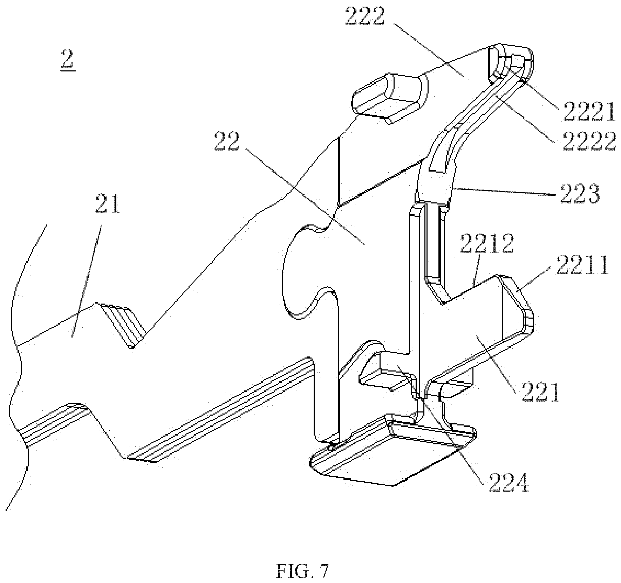

The sleeve assembly 200 is arranged on the housing, and the sleeve assembly 200 includes a propeller 2 (refer to, for example, FIG. 7 to FIG. 9B) and a sleeve sleeved on a part of the propeller 2. The stapler further includes a transmission device connected to the output shaft of the motor. The transmission device includes a first transmission mechanism and a second transmission mechanism. The sleeve and the propeller 2 are respectively connected to the motor through the first transmission mechanism and the second transmission mechanism to be driven to move along the length direction X, including moving forward or backward along the length direction X.

Referring to FIG. 1 and FIG. 2, the end effector 100 includes a jaw assembly and a staple cartridge assembly 101. The staple cartridge assembly 101 is detachably installed on the jaw assembly. The jaw assembly includes a staple cartridge holder 10 and a staple support holder 20 that is pivotably connected to the staple cartridge holder 10. The staple cartridge holder 10 is used to operably support the staple cartridge assembly 101 located therein. The staple support holder 20 can selectively move between the open position and the close position to achieve switching between the open state and the closed state, thereby clamping or releasing the tissue in conjunction with the staple cartridge holder 10 and the staple cartridge assembly 101. One end (front end) of the sleeve is connected to the staple support holder 20, and the other end (rear end) is connected to the first transmission mechanism, the first transmission mechanism can drive the sleeve to move backward or forward. The backward movement of the sleeve can allow the staple support holder 20 to pivot upwards to open the jaw assembly, while the forward movement of the sleeve can allow the staple support holder 20 to pivot downwards to close the jaw assembly.

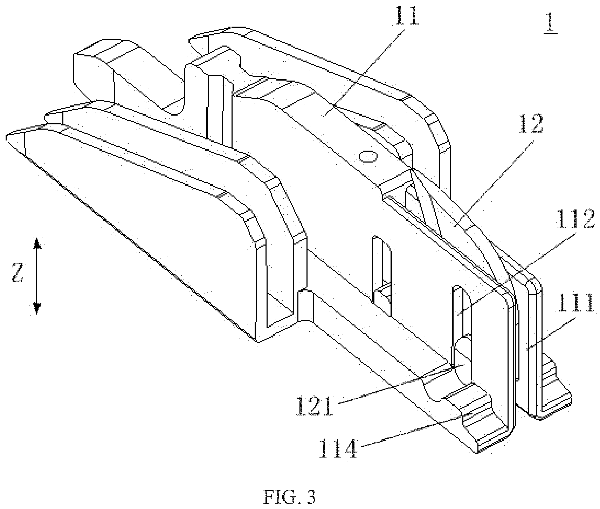

The staple cartridge assembly 101 includes a staple cartridge body 3 and a pushing staple board assembly 1. The pushing staple board assembly 1 is detachably connected to the propeller 2 to be driven by the propeller 2 to perform a strike action or a retraction action. Refer to FIG. 3 to FIG. 7, specifically, the pushing staple board assembly 1 includes a pushing staple board 11 and a cutting knife 12 movably installed on the pushing staple board 11. One end (rear end) of the propeller 2 is connected to the second transmission mechanism, and the other end (front end) is detachably connected to the pushing staple board 11 and the cutting knife. The part of the propeller 2 other than the other end is located inside the sleeve. During the cutting and anastomosis process, the cutting knife 12 is located in the space formed between the staple cartridge holder 10 and the staple support holder 20 of the jaw assembly. The second transmission mechanism can drive the propeller 2 to move forward and backward. When the staple cartridge assembly 101 is installed, the forward movement of the propeller 2 allows the pushing staple board assembly 1 to move forward. The pushing staple board 11 pushes the anastomosis staple out of the staple cartridge assembly 101 to fit the tissue, and the cutting knife 12 moves forward to cut the tissue.

The motor drives the jaw assembly to close to clamp the tissue through the first transmission mechanism, and then the motor drives the pushing staple board assembly 1 to move forward to cut and fit the tissue through the second transmission mechanism. Finally, the motor drives the jaw assembly to open to release the tissue through the first transmission mechanism, thereby achieving the function of cutting and anastomosis of the stapler.

The housing of the handle assembly 300 includes a handle housing and a head housing that are connected. The head housing can accommodate the transmission device, and the handle housing can be held by the user. Of course, in some embodiments, the handle housing may also accommodate some transmission devices simultaneously. In some embodiments, the handle housing can accommodate the motor, and the head housing can accommodate the aforementioned power module.

Specifically, as shown in FIG. 2, the staple cartridge assembly 101 includes a staple cartridge body 3 and a pushing staple board assembly 1. The staple cartridge assembly 101 can be installed on the staple cartridge holder 10 and can be replaceable as a consumable.

Multiple titanium staples are arranged along the length direction X inside the staple cartridge body 3, and a sliding groove extending in the length direction X is provided inside the staple cartridge body 3. The pushing staple board assembly 1 can move in the sliding groove. The pushing staple board assembly 1 is inserted into the staple cartridge body 3 from the tail of the staple cartridge body 3. When assembling a new staple cartridge assembly 101, the pushing staple board assembly 1 is inserted into the staple cartridge body 3 from the tail of the staple cartridge body 3, and a part of the pushing staple board assembly 1 is placed in the sliding groove. That is to say, the pushing staple board assembly 1 of the unused new staple cartridge assembly 101 is located at the tail of the staple cartridge assembly 101. After the staple cartridge assembly 101 is installed on the staple cartridge holder 10, push the pushing staple board assembly 1 through the propeller 2 of the sleeve assembly 200. The pushing staple board assembly 1 can slide along the length direction X of the staple cartridge body 3 towards the distal end of the staple cartridge body 3, thereby contacting and pushing out the titanium staples in the staple cartridge body 3. The titanium staple is pushed out, i.e., the stapler performs the anastomosis action. Specifically, the anastomosis action is achieved by the front end of the pushing staple board 11 contacting the titanium staple and pushing the titanium staple out.

The staple cartridge assembly 101 further includes a cutting knife 12. Specifically, the pushing staple board assembly 1 includes a cutting knife 12 movably installed on the pushing staple board 11. The cutting knife 12 can selectively move between the first position and the second position to allow the cutting knife 12 to switch between the hidden state and blade protrusion state. The hidden state (i.e., the cutting knife 12 is hidden below the staple outlet surface 31 of the staple cartridge body 3) can prevent users from being accidentally injured by the blade. When the staple cartridge assembly 101 is not installed on the staple cartridge holder 10, the cutting knife 12 is in the hidden state; The blade protrusion state (i.e., the blade of the cutting knife 12 protrudes out of the staple outlet surface 31 of the staple cartridge body 3) allows the blade to cut the tissue clamped by the jaw assembly. When the staple cartridge assembly 101 is installed on the staple cartridge holder 10 and performs cutting, the cutting knife 12 is in the blade protrusion state. When cutting the tissue clamped by the jaw assembly, the staple outlet surface 31 contacts with the clamped tissue.

When the stapler performs the anastomosis action, the cutting knife 12 is in the blade protrusion state. As the pushing staple board 11 moves forward, the cutting knife 12 also slides along the length direction X of the staple cartridge body 3 towards the distal end of the staple cartridge body 3, thereby cutting the tissue, that is, the stapler performs the cutting action.

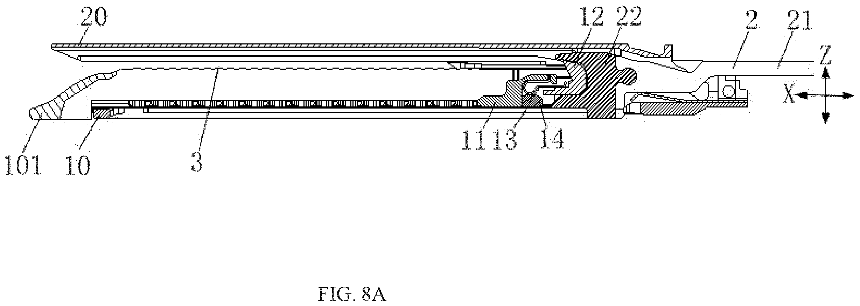

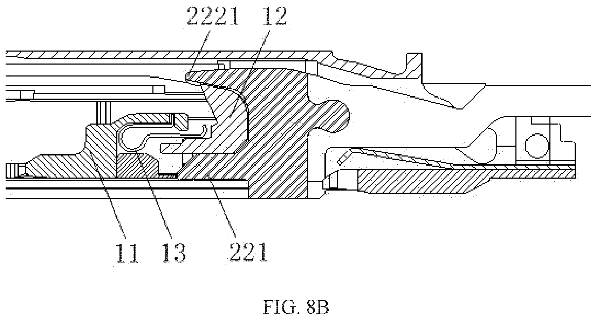

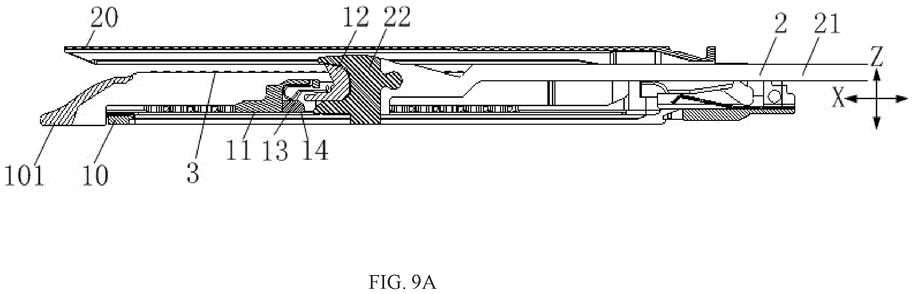

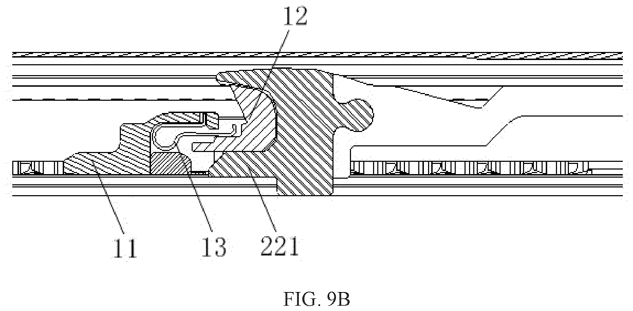

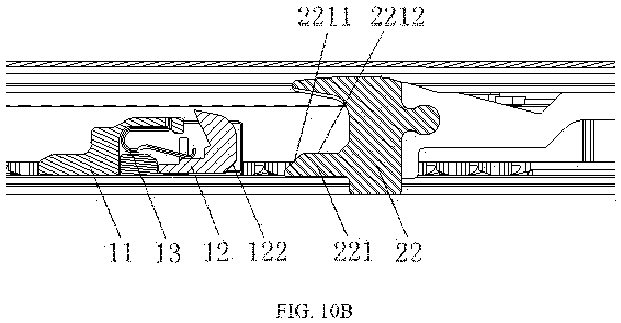

FIG. 3 and FIG. 10B show the state of cutting knife 12 in the first position, i.e., the hidden state. FIG. 8A, FIG. 8B, FIG. 9A, and FIG. 9B show the state of cutting knife 12 in the second position, i.e., the blade protrusion state.

As shown in FIG. 3 to FIG. 6, the pushing staple board assembly 1 includes a pushing staple board 11, a cutting knife 12, an elastic body 13, and a support board 14. The pushing staple board assembly 1 in this application realizes the blade hiding and protrusion of the cutting knife 12.

The pushing staple board 11 is provided with a knife receiving groove 110 that can accommodate at least a part of the cutting knife 12. The knife receiving groove 110 is provided with a first guiding member 112, and the cutting knife 12 is provided with a second guiding member 121. One of the first guiding member 112 and the second guiding member 121 extends along the direction perpendicular to the staple outlet surface 31 of the staple cartridge body 3. The first guiding member 112 and the second guiding member 121 can be connected in a relatively movable manner, and the first guiding member 112 and the second guiding member 121 can guide the cutting knife 12 to move along the direction perpendicular to the staple outlet surface 31.

In a specific implementation, as shown in FIG. 3 to FIG. 6, the first guiding member 112 may be a guiding groove extending along the height direction Z of the staple cartridge assembly 101. The knife receiving groove 110 includes a slot 111 and the following accommodating chamber. The pushing staple board 11 is provided with two opposite side walls, and these two opposite side walls and the space between these two opposite side walls form the slot 111. The slot 111 includes the two opposite side walls and the space between the two side walls. The first guiding member 112 is arranged in the slot 111 of the knife receiving groove 110. Specifically, the first guiding member 112 is arranged on the side wall of the slot 111. The second guiding member 121 may be a protrusion block protruding from the side of the cutting knife 12. By embedding the protrusion block into the guiding groove, the cutting knife 12 can be at least partially accommodated in the knife receiving groove 110. With the guidance of the guiding groove, the cutting knife 12 can move between the first position and the second position along the height direction Z (the direction perpendicular to the staple outlet surface 31) of the staple cartridge assembly 101. When the protrusion block is located at the bottom of the guiding groove, the cutting knife 12 is in the first position, and when the protrusion block is located at the top of the guiding groove, the cutting knife 12 is in the second position.

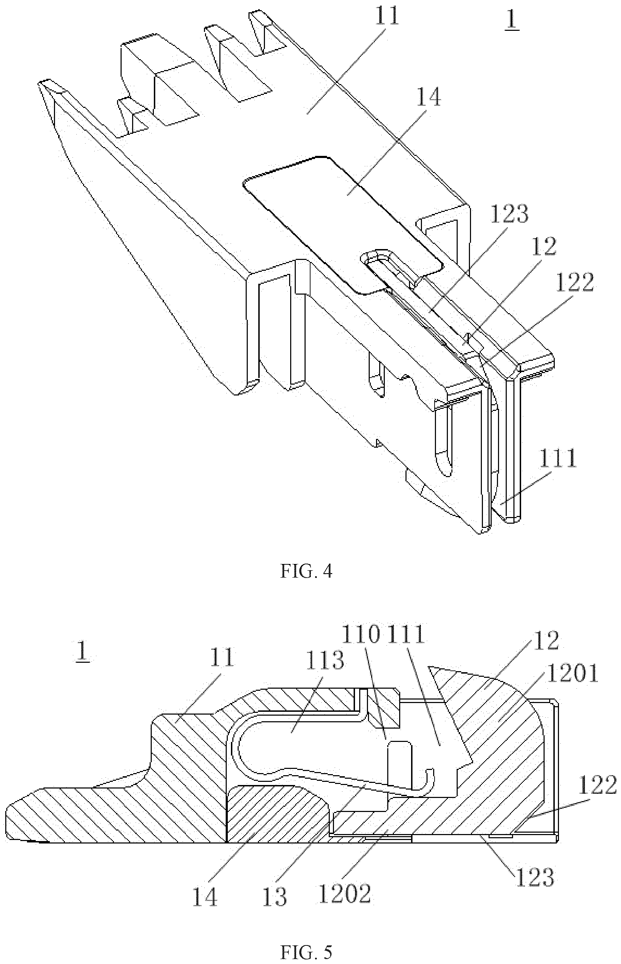

Three sides of the slot 111 are provided with openings, and the openings are located on the upper, lower, and rear sides of the pushing staple board 11. The opening on the upper side is an opening facing upwards, and the opening on the lower side is an opening facing downwards. The cutting knife 12 can be installed into the knife receiving groove 110 through the lower and rear sides of the pushing staple board 11. The first surface 123 of the cutting knife 12 is exposed from the opening, facing downwards, of the slot 111, which enables the propeller 2 to contact the cutting knife 12 from the opening on the lower side of the slot 111 and push the cutting knife 12 to move upwards. When the cutting knife 12 moves upwards, the blade protrudes out through the opening on the upper side of the slot 111, and the opening on the rear side of the slot 111 will not hinder the upward movement of the cutting knife 12. The side wall of the slot 111 is thin and elastic. When installing the cutting knife 12, the side wall of the slot 111 can be elastically opened to allow the second guiding member 121 to match with the first guiding member 112 of the cutting knife 12. The cutting knife 12 includes a knife body 1201 and a retaining structure 1202. The knife body includes a blade. As shown in FIG. 5, the knife body is partially accommodated in the slot 111, a part of the retaining structure is accommodated in the slot 111, and other part of the remaining retaining structure extends into the accommodating chamber 113 and is accommodated in the accommodating chamber 113. In another embodiment, based on the content shown in FIG. 5, the rear end of the knife body is exposed outside the slot 111.

In other embodiments, in the hidden state, the knife body 1201 of the cutting knife 12 is fully accommodated in the slot 111, which does not affect the implementation of the present application.

In other possible implementation, the first guiding member 112 may be a protrusion block, and the second guiding member 121 may be a guiding groove. Alternatively, the first guiding member 112 may be a sliding block or a sliding rail, and the second guiding member 121 may be a sliding rail or a sliding block. Those skilled in the art can understand that the implementations that can achieve guiding functions all fall within the protection scope of the present application.

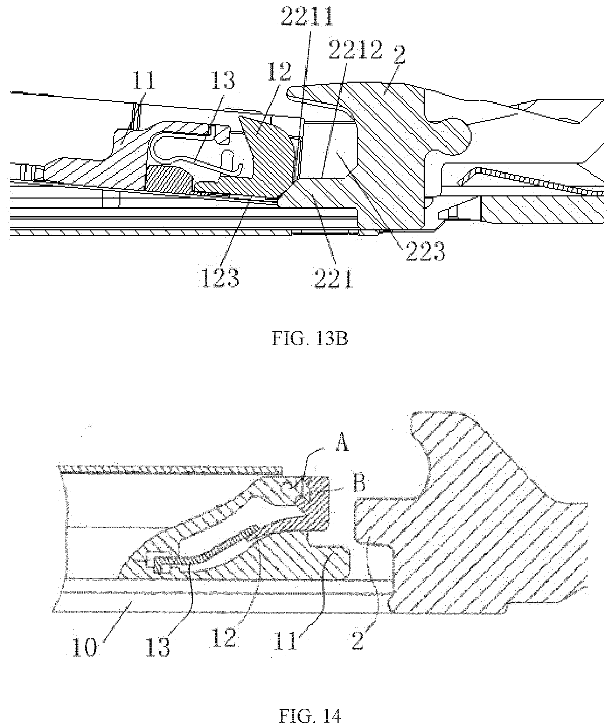

FIG. 14 shows a possible staple cartridge assembly 101 with an inclined blade protrusion. The cutting knife 12 can be installed on the pushing staple board 11 slidably relative to the pushing staple board 11. The pushing staple board 11 is provided with a guiding groove A, which tilts and extends forward. The cutting knife 12 is provided with a sliding block B, which can slide along the guiding groove A. When the propeller 2 moves forward (the left side in the figure), the propeller 2 contacts and pushes the cutting knife 12 from the rear end of the cutting knife 12. The cutting knife 12 can move diagonally upward relative to the pushing staple board 11 under the guidance of the guiding groove A, so as to allow the cutting knife 12 to achieve blade protrusion. When the blade protrusion is completed, the cutting knife 12 moves forward relative to the pushing staple board 11 by a fixed distance C, and the fixed distance C is equal to the length of the guiding groove A along the front and rear direction.

In one possible scenario, during blade protrusion process of the cutting knife 12, the pushing staple board 11 can remain stationary. When the blade protrusion process is completed, the displacement E that the cutting knife 12 moves forward relative to the staple cartridge holder 10 is equal to the distance that the cutting knife moves forward relative to the pushing staple board 11, that is, the length C of the guiding groove A along the front and rear direction. In another possible scenario, during the blade protrusion process of the cutting knife 12, the pushing staple board 11 may also be pushed forward by a certain distance D. When the blade protrusion process is completed, the displacement E that the cutting knife 12 moves forward relative to the staple cartridge holder 10 is the length C of the guiding groove A along the front and rear direction plus the distance D that the pushing staple board 11 moves forward.

In summary, for the staple cartridge assembly 101 with an inclined blade protrusion as shown in FIG. 14, the cutting knife 12 will move forward relative to the pushing staple board 11 by the length C of the guiding groove A in the front and rear direction when completing the blade protrusion. The design of the length of the pushing staple board 11 considers this length C. The length of the staple cartridge assembly 101 considers the pushing staple board 11, and the length of the jaw assembly of the stapler considers the staple cartridge assembly 101. Therefore, both the staple cartridge assembly 101 and the jaw assembly of the stapler will be designed to be relatively long. In addition, under normal circumstances, when the pushing staple board 11 moves forward, that is, the anastomosis staple is pushed out and starts stapling and the cutting knife is driven by the pushing staple board 11 to start cutting the tissue. However, the above-mentioned blade protrusion process does not expect the stapling and cutting, and if the stapling and cutting happens, the tissue will be incompletely cut. Therefore, in order to avoid the incomplete tissue cutting during the above process of the pushing staple board 11 moving forward by the distance D, the length design of the staple cartridge assembly also considers this distance D, resulting in longer design of the staple cartridge assembly and the jaw assembly of the stapler.

The guiding groove A and sliding block B of the staple cartridge assembly in FIG. 14 achieve the functions of the first guiding member 112 and the second guiding member 121 in the present application. The difference is that the guiding groove A and sliding block B guide the cutting knife to tilt, while the first guiding member 112 and the second guiding member 121 in the present application guide the cutting knife to move up and down. Compared to the staple cartridge assembly 101 shown in FIG. 14, during the process of the cutting knife 12 switching from the hidden state to the blade protrusion state, the first guiding member 112 and the second guiding member 121 in the present application extend along the direction perpendicular to the staple outlet surface (height direction Z), and the cutting knife 12 does not move along the front and rear direction relative to the staple cartridge holder 10. This displacement along the front and rear direction is omitted, so that the length of the pushing staple board 11 can be designed to be shorter, the staple cartridge assembly can have a shorter length in the case of maintaining the same anastomosis route, and the jaw assembly can have a shorter length, and users can allow the jaw assembly to reach narrow regions during surgery, which facilitates the operation of the stapler. The guiding assembly (the first guiding member 112 and second guiding member 121) that extends only along the height direction in the present application is the optimal method.

The guiding groove provided along the height direction Z can make the guiding groove shorter in the case that the cutting knife 12 moves the same distance along the height direction Z compared to the inclined guiding groove. The pushing staple board 11 has fewer hollow parts, which makes the structural strength of the pushing staple board 11 higher.

As shown in FIG. 4 and FIG. 5, the cutting knife 12 is provided with a first surface 123. When the staple cartridge assembly 101 is installed on the staple cartridge holder 10 of the stapler, the propeller 2 can contact the first surface 123, so that the cutting knife 12 moves upward relative to the pushing staple board 11 along the direction perpendicular to the staple outlet surface 31 (height direction Z) under the guidance of the first guiding member 112 and the second guiding member 121, so that the blade of the cutting knife 12 can protrude from the staple outlet surface 31.

Preferably, the first surface 123 is a plane parallel to the staple outlet surface 31, and the first surface 123 is parallel to the motion direction of the pushing staple board 11. This makes it easy for the cutting knife 12 to remain stable by the propeller 2 when the cutting knife 12 is lifted by the propeller 2, and it is not easy for the cutting knife 12 to slide and fall into the blade hidden state. There is no need to separately set a locking structure to prevent the cutting knife 12 from sliding and falling, making the structure of the staple cartridge assembly 101 simple. It can be understood that because the first surface 123 is parallel to the staple outlet surface 31, when the propeller 2 touches the first surface 123, it will not cause the pushing staple board 11 to be pushed forward. That is to say, during the installation process of the staple cartridge assembly 101 on the staple cartridge holder 10, the pushing staple board 11 will not move.

The rear end of the cutting knife 12 (the end near the sleeve assembly 200) is provided with a second surface 122. At least a part of the second surface 122 is a plane inclined relative to the staple outlet surface 31 or an arc-shaped surface inclined and protruding outward relative to the staple outlet surface 31. The second surface 122 faces the rear and lower sides of the pushing staple board 11. The further rear the second surface 122 is, the further upwards it extends. The second surface 122 can make the knife lifting part 221 of the propeller 2 to be easily inserted below the cutting knife 12, and through the guidance of the second surface 122, the movement of the propeller 2 in the front and rear direction can be converted into the movement of the cutting knife 12 in the up and down direction.

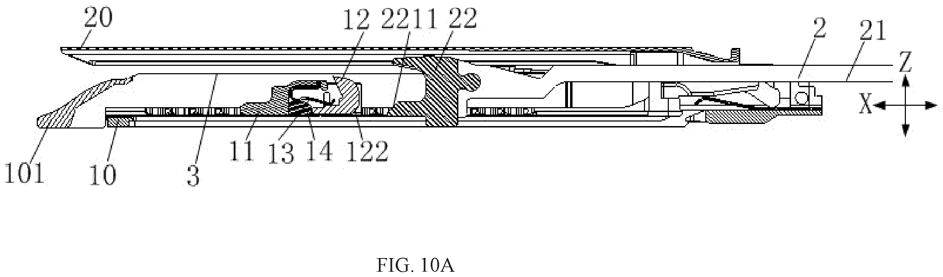

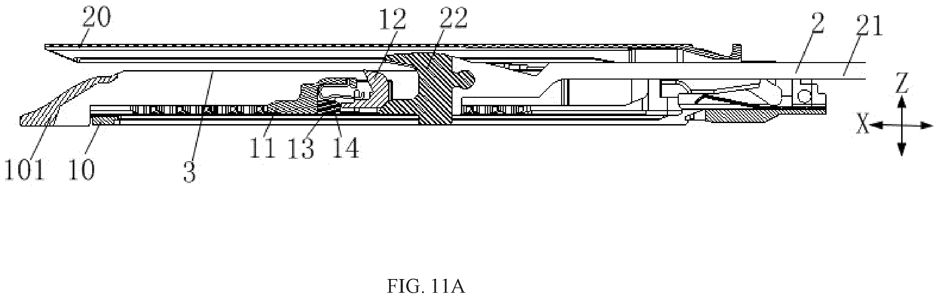

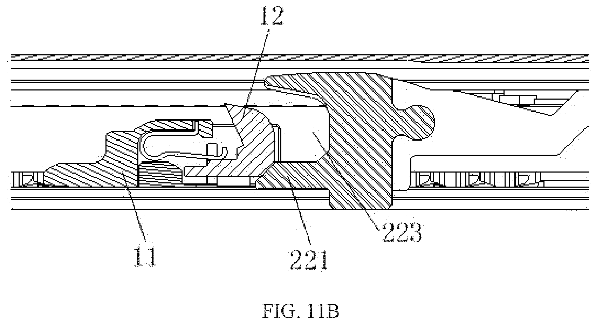

As shown in FIG. 10A, FIG. 10B, FIG. 11A, and FIG. 11B, if the stapler stops striking and retracts during the strike process, the propeller 2 will stop moving forward and retract to disengage from the cutting knife 12, thereby switching the cutting knife 12 from the blade protrusion state to the hidden state. When the stapler re-strike again, the propeller 2 moves forward again to push the cutting knife 12 and makes the cutting knife 12 to switch from the hidden state to the blade protrusion state. The process is as follows: the propeller 2 first pushes against the second surface 122, which makes the cutting knife 12 to move upwards along the first guiding member 112 against the elastic force of the elastic body 13, and makes the blade of the cutting knife 12 to protrude from the staple outlet surface 31 (upper surface) of the staple cartridge body 3, so that the blade can cut the tissue.

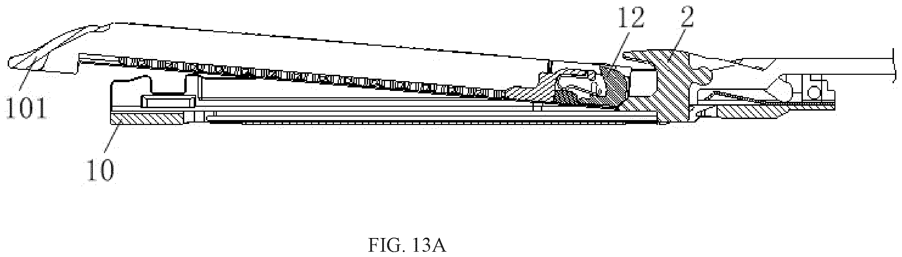

As shown in FIG. 13A and FIG. 13B, during the installation of the staple cartridge assembly 101 on the staple cartridge holder 10, the second surface 122 can be in contact with the fourth surface 2211 of the knife lifting part 221, and the staple cartridge assembly 101 can be slid towards the near end. The second surface 122 and the fourth surface 2211 can guide the cutting knife 12 to move upwards along the guiding groove to be above the knife lifting part 221.

The second surface 122 is connected with the first surface 123, and the first surface 123 is located in front of the second surface 122. The second surface 122 and the first surface 123 form at least a part of the bottom surface of the cutting knife 12. The connection between the second surface 122 and the first surface 123 can enable the two to synergize with each other. When the stapler re-strikes again after retracting the blade, the second surface 122 lifts the cutting knife 12 to achieve the blade protrusion, and the first surface 123 stabilizes the cutting knife 12 to keep the blade protrusion state. The combination of the second surface 122 and the first surface 123 enables the cutting knife 12 to achieve continuous blade protrusion state and blade protrusion maintaining state.

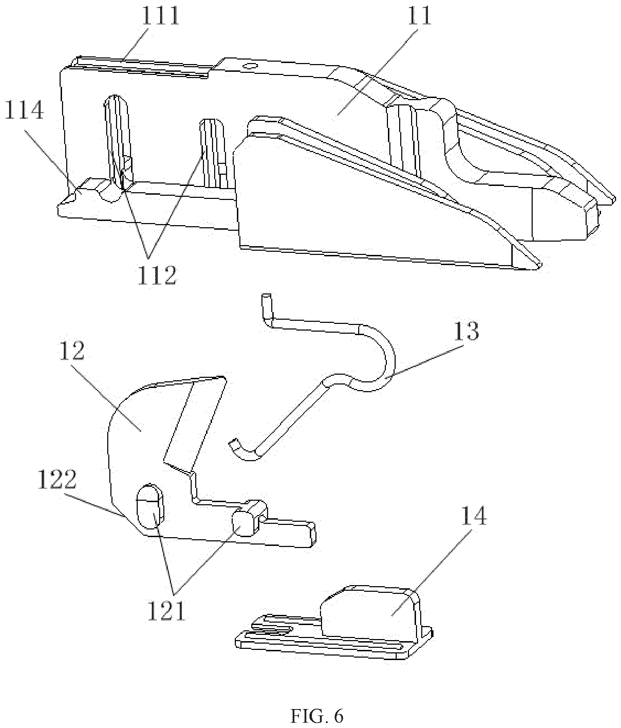

Referring to FIG. 3 and FIG. 6, the knife receiving groove 110 is provided with two sets of first guiding members 112. Here, two first guiding members 112 that are respectively located in the two side walls of the slot 111 and are opposite to each other in the width direction Y can be referred to as one set of first guiding members 112. The two sets of first guiding members 112 may be spaced apart in the length direction X, which can reliably guide the movement of the cutting knife 12. The two sets of first guiding members 112 can prevent the cutting knife 12 from overturning inside the knife receiving groove 110.

Each set of first guiding members 112 has two first guiding members 112, the two first guiding members 112 are respectively located on the two opposite side walls of the slot 111, i.e., one first guiding member 112 is located on one side wall of the slot 111, and the other first guiding member 112 is located on the other side wall opposite to the above side wall.

Corresponding to the two sets of first guiding members 112, the cutting knife 12 is provided with two sets of second guiding members 121, the two sets of second guiding members 121are spaced apart in the extension direction of the staple outlet surface 31. The front set of first guiding members 112 in the two sets of first guiding members 112 is matched with the front set of second guiding members 121 in the two sets of second guiding members 121, and the rear set of first guiding members 112 is matched with the rear set of second guiding members 121. Each set of second guiding members 121 has two second guiding members 121, the two second guiding members 121 are respectively located on the opposite sides of the cutting knife 12, that is, one second guiding member 121 is located on one side wall of the cutting knife 12, and the other second guiding member 121 is located on another side wall, opposite to the above side wall, of the cutting knife 12. Each first guiding member 112 is matched with one second guiding member 121. That is, the two first guiding members 112 of one set of first guiding members are correspondingly matched with the two second guiding members 121 of one set of second guiding members 121 in a relatively movable manner. Guiding the cutting knife 12 from two sides can prevent the cutting knife 12 from tilting left and right in the knife receiving groove 110, thereby ensuring stability during the movement of the cutting knife 12 along the height direction Z.

Furthermore, the second guiding member 121 may be a protrusion block with a tetragonum cross-section, this protrusion block is difficult to rotate in the guiding groove and can prevent the cutting knife 12 from overturning in the knife receiving groove 110. The tetragonum shape include a square, a rounded square, a rectangle, a rounded rectangle and so on.

In other embodiments, only one set of first guiding members 112 may be provided to prevent the cutting knife 12 from overturning in the knife receiving groove 110 through the shape construction of the first guiding member 112 and/or the second guiding member 121. For example, the shape of the above protrusion block serving as the second guiding member 121 is in a tetragonum shape, or the second guiding member 121 includes a locking structure for locking the positional relationship between the first guiding member 112 and the second guiding member 121.

Referring to FIG. 5, the knife receiving groove 110 includes an accommodating chamber 113, which accommodates at least a part of the elastic body 13. The elastic body 13 allows the cutting knife 12 to move downwards along the direction perpendicular to the staple outlet surface 31. The accommodating chamber 113 includes an opening, for example, the opening faces downwards. The support board 14 is installed on the pushing staple board 11 to at least partially seal the opening, thereby preventing the cut human tissue from entering from the bottom of the accommodating chamber 113, so that the movement of the elastic body 13 is not obstructed. The support board 14 may be firmly connected to the pushing staple board 11 through plastic hot melting, and is located below the elastic body 13. The support board 14 can, but does not have to, support the elastic body 13.

The width of the accommodating chamber 113 is consistent with the width of the elastic body 13, so that the accommodating chamber 113 can limit the movement range of the elastic body 13 along the width direction Y The accommodating chamber 113 is connected to the slot 111. The other parts of the retaining mechanism of the cutting knife 12 can extend into the accommodating chamber 113, while the movable end of the elastic body 13 can extend into the slot 111. One end of the elastic body 13 is installed on the pushing staple board 11, and the other end (movable end) of the elastic body 13 is pressed against the retaining mechanism of the cutting knife 12, so that the cutting knife 12 has a tendency to be stored in the knife receiving groove 110, that is, the cutting knife 12 is at the first position. The elastic body 13 may be a special-shaped spring. As shown in FIG. 5, the movable end of the elastic body 13 extends into the slot 111 and presses against the retaining mechanism of the cutting knife 12. It should be noted that the positions of the movable end of the elastic body 13 and the retaining mechanism of the cutting knife 12 in the knife receiving groove 110 are not limited to the scope of this application, as long as it is realized that the movable end of the elastic body 13 and the retaining mechanism of the cutting knife 12 are both located in the knife receiving groove 110 and the movable end of the elastic body 13 presses against the retaining mechanism of the cutting knife 12. For example, the movable end of the elastic body 13 and the retaining mechanism of the cutting knife 12 are both inside the accommodating chamber 113.

The elastic body 13 can be first installed into the accommodating chamber 113 from the bottom opening of the accommodating chamber 113, then the cutting knife 12 is installed into the slot 111, and finally the support board 14 can be installed after the cutting knife 12 and elastic body 13 are installed. The support board 14 is installed on the pushing staple board 11.

As shown in FIG. 3 and FIG. 6, the pushing staple board 11 is provided with a force bearing portion 114 for being pushed against by the propeller 2. The propeller 2 can drive the pushing staple board 11 to move forward by pushing against the force bearing portion 114.

As shown in FIG. 7, the sleeve assembly 200 includes the propeller 2, the propeller 2 includes a knife holder 21 and a knife holder head 22. The knife holder head 22 is connected to the front end of the knife holder 21, and the rear end of the knife holder 21 is connected to the second transmission mechanism. When the staple cartridge assembly 101 is installed on the staple cartridge holder 10 and the propeller 2 moves forward along the length direction X, the knife holder head 22 can push the pushing staple board 11 to move forward, and the knife holder head 22 contacts and fits with the cutting knife 12, causing the cutting knife 12 to be lifted, and the blade of the cutting knife 12 protrudes from the staple outlet surface 31 of the staple cartridge body 3.

The knife holder head 22 is provided with a pushing portion 224. When the propeller 2 moves forward, the pushing portion 224 contacts and pushes against the force bearing portion 114, thereby driving the pushing staple board 11 to move forward.

The knife holder head 22 is also provided with a knife lifting part 221 and an arm portion 222, the knife lifting part 221 and the arm portion 222 extend forward and protrude forward. The knife lifting part 221 and the arm portion 222 are spaced apart, and a concave part 223 which can accommodate the cutting knife 12 is formed between the knife lifting part 221 and the arm portion 222. The cutting knife 12 can be sandwiched between the knife lifting part 221 and the arm portion 222. In the state where the cutting knife 12 is accommodated in the concave part 223, the knife lifting part 221 is located below the cutting knife 12, and the arm portion 222 is located above the cutting knife 12. The cutting knife 12 may not contact the bottom surface of the concave part 223.

The knife lifting part 221 is provided with a third surface 2212, the third surface 2212 contacts with the first surface 123 (bottom surface) of the cutting knife 12, thereby keeping the cutting knife 12 in a lifted state. Preferably, in a state that the staple cartridge assembly 101 is installed on the staple cartridge holder 10, the third surface 2212 is parallel to the staple outlet surface 31, the third surface 2212 is parallel to the first surface 123 of the cutting knife 12, and the third surface 2212 is parallel to the motion direction (front and rear direction) of the pushing staple board 11. When the cutting knife 12 remains in the blade protrusion state and the propeller 2 is stationary, the knife lifting part 221 contacts the cutting knife 12. The knife lifting part 221 does not apply an external force along the length direction X to the cutting knife 12, so that the cutting knife 12 can remain stationary. Thus, it is easy for the cutting knife 12 to remain stable when lifted by the propeller 2, and it is not easy for the cutting knife 12 to slide into the hidden state. There is no need to separately set a locking structure to prevent the cutting knife 12 from sliding, thereby making the structure of the staple cartridge assembly 101 simple.

The front end of the knife lifting part 221 is provided with a fourth surface 2211. The fourth surface 2211 is a flat surface that is inclined relative to the staple outlet surface 31 of the staple cartridge assembly 101 installed on the jaw assembly or an arc-shaped surface that is inclined and concave inward relative to the staple outlet surface 31. The fourth surface 2211 faces the front and upper sides of the stapler. The further rear the fourth surface 2211 is, the further upwards it extends. When the staple cartridge assembly 101 is installed on the staple cartridge holder 10, the fourth surface 2211 and the second surface 122 can be parallel, so that when the second surface 122 and the fourth surface 2211 are matched, the contact area can be larger and wear can be reduced.

As shown in FIG. 10A to FIG. 11B, when the propeller 2 pushes the cutting knife 12 to move forward to make the cutting knife 12 to realize blade protrusion, the fourth surface 2211 and the second surface 122 come into contact. As the knife lifting part 221 moves forward, the second surface 122 and the fourth surface 2211 gradually stagger, causing the cutting knife 12 to overcome the elastic force of the elastic body 13 and move upwards along the first guiding member 112. The cutting knife 12 is accommodated in the concave part 223, causing the blade of the cutting knife 12 to protrude from the staple outlet surface 31 of the staple cartridge body 3.

In other possible embodiments, only the cutting knife 12 may be provided with a second surface 122 that is inclined, or only the front end of the knife lifting part 221 may be provided with a fourth surface 2211 that is inclined. When the knife lifting part 221 moves forward, the second surface 122 or the fourth surface 2211 can cause the knife lifting part 221 to push the cutting knife 12 to move upwards. Preferably, the other one of the front end of the knife lifting part 221 or the cutting knife 12 is provided with a rounded corner that matches the inclined surface.

As shown in FIG. 8B and FIG. 9B, when the cutting knife 12 is accommodated in the concave part 223, the front end of the arm portion 222 protrudes forward from the tip of the cutting knife 12. A guiding surface 2221 is formed at the front end of the arm portion 222, the guiding surface 2221 faces the concave part 223. The guiding surface 2221 may be an inclined surface or an arc-shaped surface. When the knife holder head 22 pushes the cutting knife 12 to move forward, the guiding surface 2221 comes into contact with the tissue before the cutting knife 12, thereby guiding the tissue to move towards the blade of the cutting knife 12, making it easier for the tissue to be cut by the cutting knife 12. The tip of the cutting knife 12 can be the tip of the blade of the cutting knife 12, or the tip of the cutting knife 12 may be the front end of the exposed part of the cutting knife 12. In some possible embodiments, the tip of the cutting knife 12 can be either the tip of the blade or not the tip of the blade.

Referring to FIG. 7 to FIG. 9B, the arm portion 222 is provided with a groove 2222, the groove 2222 faces the concave part 223. The groove 2222 extends substantially along the length direction X. The groove 2222 may be provided in the middle position of the arm portion 222. The surface towards the concave part 223 defining the side wall of the groove 2222 is a guiding surface 2221. When the cutting knife 12 is accommodated in the concave part 223, the upper edge of the cutting knife 12 can be embedded in the groove 2222. In this way, there will be no gap in the height direction between the cutting knife 12 and the arm portion 222 as a whole, preventing tissue from entering the gap between the cutting knife 12 and the arm portion 222 and not being cut, and ensuring that all tissue can be cut by the cutting knife 12 with a better cutting effect.

Before the stapler first cuts the tissue and cuts into the tissue, the cutting knife 12 needs to be switched from the hidden state to the blade protrusion state. In this application, through the structure of the knife lifting part 221 mentioned above, the doctor can achieve complete blade protrusion of the cutting knife 12 only through installation action, without the need for the doctor to activate the stapler to begin striking. Specifically, when the staple cartridge assembly 101 of this application is installed obliquely on the jaw assembly, the following two situations may occur.

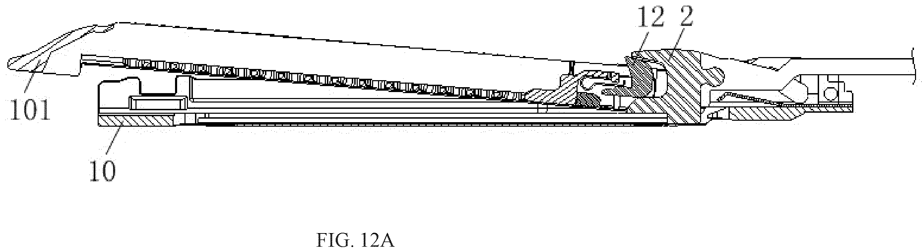

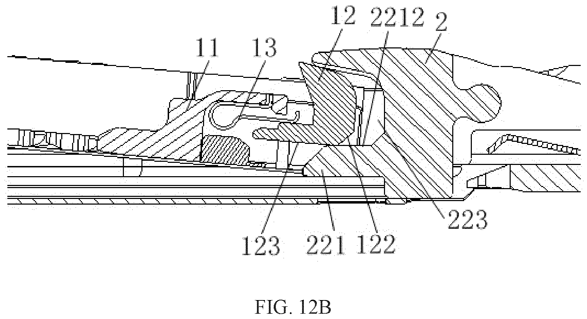

Situation 1: as shown in FIG. 12A and FIG. 12B, when installing the staple cartridge assembly 101, the distal end of the staple cartridge assembly 101 is lifted upwards and forms an inclined angle. The near end of the staple cartridge assembly 101 first extends into the near end of the jaw assembly, and the near cutting knife 12 is partially accommodated in the concave part 223 of the propeller 2. In this case, the first surface 123 of the cutting knife 12 rests against the third surface 2212 of the knife lifting part 221. The installation action causes the first surface 123 of the cutting knife 12 to receive an upward lifting force, thereby causing the cutting knife 12 to be lifted by the knife lifting part 221 and achieve the blade protrusion. Finally, the cutting knife 12 is lifted to the second position. Then, as the staple cartridge assembly 101 continues to slide and extends towards the near end of the jaw assembly, the third surface 2212 and the first surface 123 of the cutting knife 12 slide relative to each other until the cutting knife 12 is embedded in the concave part 223. Finally, the distal end of the staple cartridge assembly 101 is pressed downward, and when pressed to the end, the third surface 2212 stabilizes the cutting knife 12 in the second position, i.e., the staple cartridge assembly 101 is installed on the staple cartridge holder 10.

Situation 2: as shown in FIG. 13A and FIG. 13B, when installing the staple cartridge assembly 101, the distal end of the staple cartridge assembly 101 is lifted upwards, and forms an inclined angle. The near end of the staple cartridge assembly 101 first extends into the near end of the jaw assembly, but the cutting knife 12 is not directly embedded in the concave part 223 of the propeller 2, but located on the front side of the knife lifting part 221 or in contact with the fourth surface 2211 of the knife lifting part 221. The doctor operates the staple cartridge assembly 101 to continue to slide towards the near end of the jaw assembly. In this case, the second surface 122 of the cutting knife 12 at the near end of the staple cartridge assembly 101 rests against the fourth surface 2211 of the knife lifting part 221, then the contact surface becomes smaller and the surfaces are gradually staggered, finally detached. During this process, under the guidance of these two surfaces, the cutting knife 12 can be lifted by the knife lifting part 221 to perform the blade protrusion, and finally the cutting knife 12 is lifted to the second position. When the above detachment occurs, the cutting knife 12 enters the concave part 223 of the knife lifting part. As shown in FIG. 12A and FIG. 12B, the third surface 2212 of the knife lifting part is against the first surface 123 of the cutting knife 12 while keeping the cutting knife 12 in the second position. As the staple cartridge assembly 101 continues to slide and extend towards the near end of the jaw assembly, the third surface 2212 and the first surface 123 of the cutting knife 12 slide relative to each other until the cutting knife 12 is embedded in the concave part 223. Finally, the distal end of the staple cartridge assembly 101 is pressed downward, and when pressed to the end, the third surface 2212 stabilizes the cutting knife 12 in the second position, i.e., the staple cartridge assembly 101 is installed on the staple cartridge holder 10. Through the guidance of the second surface 122 and the fourth surface 2211, the staple cartridge assembly 101 is installed in the predetermined position and is less likely to be installed in misaligned position.

In above two installation methods, in the state where the knife holder head 22 is in the starting position, the cutting knife 12 is lifted by the knife lifting part 221, so that the blade of the cutting knife 12 protrudes from the staple outlet surface 31 of the staple cartridge body 3, completing the blade protrusion. Both of the above installation methods can avoid displacement of the installation position of the staple cartridge assembly 101, thereby avoiding damage to the pushing staple board 11 when the jaws are closed and reducing surgical risks.

In the above installation method, it can be seen that when the staple cartridge assembly 101 is installed on the jaw assembly, the knife lifting part 221 is located below the cutting knife 12, and the third surface 2212 acts on the cutting knife 12 from below the cutting knife 12, causing the blade of the cutting knife 12 to protrude from the staple outlet surface 31 of the staple cartridge assembly 101. The term "act on" here includes pushing; the third surface 2212 pushes the cutting knife 12 from below the cutting knife 12, causing the cutting knife 12 to move in the direction perpendicular to the staple outlet surface 31, so that the blade of the cutting knife 12 protrudes from the staple outlet surface 31 of the staple cartridge assembly 101. The term "act on" also includes contacting; the third surface 2212 contacts the cutting knife 12 from below the cutting knife 12 to maintain the blade of the cutting knife 12 protruding from the staple outlet surface 31.

Refer to FIG. 8A to FIG. 11B to illustrate the working process of the propeller 2 pushing the pushing staple board assembly 1 in a state where the staple cartridge assembly 101 is installed on the staple cartridge holder 10.

As shown in FIG. 8A and FIG. 8B, the staple cartridge assembly 101 is installed on the staple cartridge holder 10, and the pushing staple board assembly 1 is located at the rear end of the staple cartridge assembly 101 in the starting position. The cutting knife 12 is lifted by the knife lifting part 221 and achieve the blade protrusion.

As shown in FIG. 9A and FIG. 9B, the knife holder head 22 moves forward from the starting position to a certain position in the middle of the staple cartridge body 3. During this process, the knife lifting part 221 keeps the cutting knife 12 lifted. The knife holder head 22 pushes the pushing staple board 11 and the cutting knife 12 to move forward, and the pushing staple board 11 pushes the anastomosis staple to achieve stapling to match the tissue, and the blade of the cutting knife 12 can cut the tissue.

As shown in FIG. 10A and FIG. 10B, when the striking stops and the blade is retracted, the knife holder head 22 moves backward a certain distance from a certain position in the middle, causing the knife holder head 22 to separate from the pushing staple board assembly 1. As the cutting knife 12 is limited by the first guiding member 112, the knife lifting part 221 also separates from the cutting knife 12. Due to the retraction of the knife lifting part 221 below the cutting knife 12, the cutting knife 12 moves downward under the elastic force of the elastic body 13. The blade of the cutting knife 12 is lower than the staple outlet surface 31 of the staple cartridge body 3 and is in a hidden state. During the retraction process of the knife holder head 22, the position of the cutting knife 12 along the length direction X remains unchanged to avoid the problem of cutting knife 12 jamming caused by the retraction of the cutting knife 12 along with the knife holder head 22.

As shown in FIG. 11A and FIG. 11B, when the knife holder head 22 moves forward again after knife retraction, the fourth surface 2211 of the knife lifting part 221 first contacts the second surface 122 of the cutting knife 12. As the knife lifting part 221 moves forward, the contact surface between the second surface 122 and the fourth surface 2211 becomes smaller and the surfaces are gradually staggered, causing the cutting knife 12 to overcome the elastic force of the elastic body 13 and move upwards along the first guiding member 112. When the second surface 122 and the fourth surface 2211 are completely staggered, the third surface 2212 of the knife lifting part 221 rests against the first surface 123 of the cutting knife 12, causing the cutting knife 12 to remain lifted. The third surface 2212 and the first surface 123 of the cutting knife 12 slide relative to each other until the cutting knife 12 is embedded in the concave part 223, causing the blade of the cutting knife 12 to protrude from the staple outlet surface 31 of the staple cartridge body 3, and allowing the cutting knife 12 to cut the tissue.

The surgical instrument in this application is capable of simultaneously performing anastomosis and cutting, the surgical instrument includes but not limited to the stapler, suturing equipment, and surgical robot, all of which have end effectors and propellers. Although this application provides a specific description of the surgical instrument being a stapler as an example, the surgical instrument in this application is not limited to the stapler.

Although the present application is described in detail using the implementations described above, it is apparent to those skilled in the art that the present application is not limited to the implementations described in this specification. This application can be implemented by being modified and implemented to form modified embodiments without departing from the spirit and scope determined by the claims. Therefore, the records in this specification are for illustrative purposes and do not have any restrictive meaning for the present application.

## Claims

1. A staple cartridge assembly (101), comprising a staple cartridge body (3) and a pushing staple board assembly (1) installed on the staple cartridge body (3), wherein the pushing staple board assembly (1) comprises:
a pushing staple board (11), wherein the pushing staple board (11) is provided with a knife receiving groove (110), the knife receiving groove (110) is provided with a first guiding member (112), and
a cutting knife (12), wherein the cutting knife (12) is at least partially accommodated in the knife receiving groove (110), and the cutting knife (12) is provided with a second guiding member (121);
one of the first guiding member (112) and the second guiding member (121) extends along a direction perpendicular to a staple outlet surface (31) of the staple cartridge body (3), the first guiding member (112) and the second guiding member (121) are connected in a relatively movable manner, and the first guiding member (112) and the second guiding member (121) are capable of guiding the cutting knife (12) to move along a direction perpendicular to the staple outlet surface (31).

2. The staple cartridge assembly according to claim 1, wherein the cutting knife (12) comprises a first surface (123), the first surface (123) is configured to be pushed to allow the cutting knife (12) to move upwards relative to the pushing staple board (11) along the direction perpendicular to the staple outlet surface (31) under a guidance of the first guiding member (112) and the second guiding member (121), to allow a blade of the cutting knife (12) to protrude from the staple outlet surface (31).

3. The staple cartridge assembly according to claim 2, wherein after the cutting knife (12) moves upward in the direction perpendicular to the staple outlet surface (31) to allow the blade of the cutting knife (12) to protrude from the staple outlet surface (31), the first surface (123) is contacted to maintain the blade of the cutting knife (12) protruding from the staple outlet surface (31).

4. The staple cartridge assembly according to any one of claims 2 to 3, wherein the first surface (123) is a plane parallel to the staple outlet surface (31).

5. The staple cartridge assembly according to claim 2, wherein the knife receiving groove (110) is provided with an opening, the opening allows the first surface (123) to be exposed from the knife receiving groove (110) and to be contactable.

6. The staple cartridge assembly according to claim 1, wherein the cutting knife (12) comprises a second surface (122), at least a part of the second surface (122) is a plane inclined relative to the staple outlet surface (31) or an arc-shaped surface inclined and protruding outward relative to the staple outlet surface (31), the second surface (122) faces rear and lower sides of the pushing staple board (11), the second surface (122) is configured to be pushed to allow the cutting knife (12) to move upwards in the direction perpendicular to the staple outlet surface (31) under a guidance of the first guiding member (112) and the second guiding member (121), to allow a blade of the cutting knife (12) to protrude from the staple outlet surface (31).

7. The staple cartridge assembly according to claim 6, wherein a first surface (123) of the cutting knife (12) is connected with the second surface (122), and the first surface (123) is located on a front side of the second surface (122); the first surface (123) is contacted to maintain the blade of the cutting knife (12) protruding from the staple outlet surface (31).

8. The staple cartridge assembly according to claim 7, wherein the first surface (123) is a plane parallel to the staple outlet surface (31).

9. The staple cartridge assembly according to claim 2, wherein the cutting knife (12) further comprises a second surface (122), at least a part of the second surface (122) is a surface inclined relative to the staple outlet surface (31) or an arc-shaped surface inclined and protruding outward relative to the staple outlet surface (31), the second surface (122) faces rear and lower sides of the pushing staple board (11), the second surface (122) is configured to be pushed to allow the cutting knife (12) to move upwards in the direction perpendicular to the staple outlet surface (31) under a guidance of the first guiding member (112) and the second guiding member (121), to allow the blade of the cutting knife (12) to protrude from the staple outlet surface (31);
the first surface (123) is connected with the second surface (122), and the first surface (123) is located on a front side of the second surface (122).

10. The staple cartridge assembly according to claim 1, wherein the knife receiving groove (110) is provided with at least one set of first guiding members (112), and one set of first guide members (112) comprises two first guiding members (112) located respectively on opposite side walls of the knife receiving groove (110), the cutting knife (12) is provided with at least one set of second guide members (121), one set of second guide members (121) comprises two second guiding members (121) located respectively on opposite sides of the cutting knife (12), the two first guiding members (112) of the one set of first guiding members (112) are matched with the two second guiding members (121) of the one set of second guiding members (121) one by one.

11. The staple cartridge assembly according to claim 9, wherein the knife receiving groove (110) is provided with two sets of first guiding members (112), the two sets of first guiding members (112) are spaced apart in an extension direction of the staple outlet surface (31), the cutting knife (12) is provided with two sets of second guiding members (121), the two sets of second guiding members (121) are spaced apart in the extension direction of the staple outlet surface (31), a front set of first guiding members (112) in the two sets of first guiding members (112) is matched with a front set of second guiding members (121) in the two sets of second guiding members (121), and a rear set of first guiding members (112) in the two sets of first guiding members (112) is matched with a rear set of second guiding members (121) in the two sets of second guiding members (121).

12. The staple cartridge assembly according to claim 1, wherein the pushing staple board assembly (1) further comprises an elastic body (13), one end of the elastic body (13) is installed on the pushing staple board (11), the other end of the elastic body (13) is against the cutting knife (12), and the elastic body (13) is configured to drive the cutting knife (12) to move downwards in the direction perpendicular to the staple outlet surface (31).

13. The staple cartridge assembly according to claim 11, wherein the pushing staple board assembly (1) further comprises a support board (14), the knife receiving groove (110) comprises an accommodating chamber (113) for accommodating at least a part of the elastic body (13), the accommodating chamber (113) comprises an opening, and the support board (14) is installed on the pushing staple board (11) to at least partially seal the opening.

14. A surgical instrument, comprising an end effector (100) and a propeller (2), wherein the end effector (100) comprises a jaw assembly and the staple cartridge assembly (101) as claimed in any one of claims 1 to 12, the staple cartridge assembly (101) is detachably installed on the jaw assembly,
the propeller (2) is configured to drive the pushing staple board (11) to move forward along a length direction (X), the propeller (2) comprises a knife lifting part (221) extending forward, the knife lifting part (221) comprises a third surface (2212);
in a case that the staple cartridge assembly (101) is installed on the jaw assembly, the knife lifting part (221) is located below the cutting knife (12), and the third surface (2212) acts on the cutting knife (12) from below the cutting knife (12), to allow a blade of the cutting knife (12) to protrude from the staple outlet surface (31) of the staple cartridge assembly (101).

15. The surgical instrument according to claim 14, wherein the third surface (2212) is a plane parallel to the staple outlet surface (31) of the staple cartridge assembly (101) installed on the jaw assembly.

16. The surgical instrument according to claim 14, wherein the knife lifting part (221) further comprises a fourth surface (2211), the fourth surface (2211) is a surface inclined relative to the staple outlet surface (31) of the staple cartridge assembly (101) installed on the jaw assembly, or an arc-shaped surface inclined and recessed inwardly relative to the staple outlet surface (31), the fourth surface (2211) faces front and upper sides of the surgical instrument;
in a case that the knife lifting part (221) moves forward, the fourth surface (2211) is capable of contacting and pushing the cutting knife (12) to slide relative to the fourth surface (2211), thereby allowing the cutting knife (12) to move upward in the direction perpendicular to the staple outlet surface (31).

17. The surgical instrument according to claim 16, wherein the third surface (2212) is connected with the fourth surface (2211), and the fourth surface (2211) is located on a front side of the third surface (2212).

18. The surgical instrument according to claim 14, wherein the propeller (2) is provided with an arm portion (222), wherein the arm portion (222) and the knife lifting part (221) are spaced apart, and a concave part (223) is provided between the arm portion (222) and the knife lifting part (221), the concave part (223) is capable of accommodating the cutting knife (12).

19. The surgical instrument according to claim 18, wherein a guiding surface (2221) is provided at a front end of the arm portion (222), in a case that the cutting knife (12) is accommodated in the concave part (223), the guiding surface (2221) protrudes forward from a tip of the cutting knife (12), the guiding surface (2221) faces the concave part (223), and the guiding surface (2221) is an inclined surface or an arc-shaped surface.

20. The surgical instrument according to claim 18, wherein the arm portion (222) is provided with a groove (2222), the groove (2222) faces the concave part (223), in a case that the cutting knife (12) is accommodated in the concave part (223), an upper edge of the cutting knife (12) is embedded in the groove (2222).

21. The surgical instrument according to claim 14, wherein the third surface (2212) is configured to push the cutting knife (12) from below the cutting knife (12), to allow the cutting knife (12) to move in the direction perpendicular to the staple outlet surface (31), so that the blade of the cutting knife (12) protrudes from the staple outlet surface (31) of the staple cartridge assembly (101); or
the third surface (2212) contacts the cutting knife (12) from below the cutting knife (12) to maintain the blade of the cutting knife (12) protruding from the staple outlet surface (31).
